# EUROPÄISCHE PATENTSCHRIFT

(11) **EP 3 986 350 B1**
(45) Veröffentlichungstag und Bekanntmachung des Hinweises auf die Patenterteilung: **12.07.2023**
(21) Anmeldenummer: 20796702.7
(22) Anmeldetag: 07.10.2020
(51) Int. Cl.: A61F 13/42

(54) **VORRICHTUNG ZUR ÜBERWACHUNG DES ZUSTANDES EINER WINDEL**
DEVICE FOR MONITORING THE STATUS OF A NAPPY
DISPOSITIF DE SURVEILLANCE DE L'ÉTAT D'UNE COUCHE

(30) Priorität: 08.10.2019 DE 102019126991
(43) Veröffentlichungstag der Anmeldung: 27.04.2022
(73) Patentinhaber: mySenti GmbH, 50765 Köln (DE)
(72) Erfinder: HERWEG, Manuel, 50765 Köln (DE)
(74) Vertreter: Kohlmann, Kai
(86) Internationale Anmeldenummer: PCT/EP2020/078034
(87) Internationale Veröffentlichungsnummer: WO 2021/069461

(56) Entgegenhaltungen:
- EP-A1- 1 082 711
- EP-A1- 3 478 236
- DE-A1-102013 102 228
- US-A- 5 959 535
- US-A1- 2008 284 608

## Beschreibung

Die Erfindung betrifft eine Vorrichtung zur Überwachung des Zustandes einer Einwegwindel mit einer saugfähiges Material zur Aufnahme von Urin oder Stuhl umfassenden Außenhülle, insbesondere in der Säuglings- und Altenpflege.

Derartige Vorrichtungen - auch als Windelsensoren bezeichnet - helfen, Hautschäden oder Infektionen beim Träger der Windel zu vermeiden. Zudem wird der Schlaf des Trägers verbessert. Des Weiteren unterstützen Windelsensoren einen Windelwechsel nur dann durchzuführen, wenn er auch tatsächlich notwendig ist.

Unter der Bezeichnung CURALUNA ist bereits ein Windelsensor bekannt geworden, der fortlaufend ermittelt, wann eine Windel gewechselt werden muss. Der wiederverwendbare Windelsensor misst die Feuchtigkeit der Windel und wird per Klettverschluss auf handelsüblichen Einwegwindeln an der Außenseite angebracht. Die Signalisierung, ob ein Windelwechsel erforderlich ist, erfolgt optisch und/oder akustisch. Hierzu werden die Daten über eine dazugehörige App an ein Mobiltelefon übertragen. Der Sensor befindet sich in einem Gehäuse und kann falls notwendig gereinigt werden (Abgerufen unter http:\\www.curaluna.de am 11.09.2020).

Die EP 3 158 980 A1 offenbart einen Sensor, der entweder in eine Windel fest integriert ist oder auch als Einlage in der Windel verwendbar ist. An den Sensor ist eine Elektronik angeschlossen, die via Bluetooth Daten auf ein Smartphone oder ein anderes Bluetooth fähiges Gerät übertragen kann. Ist eine Verunreinigung einer Windel erfolgt, kann die Elektronik zur Auswertung der Sensorsignale einfach entfernt werden und an einen Sensor in einer neuen Windel bzw. Einlage angeschlossen werden. Somit wird nur die Windel einschließlich Sensor erneuert, nicht jedoch die Elektronik.

Die CN 10297208 A offenbart einen Windelsensor umfassend einen Sensor zur Erfassung der Temperatur und Feuchtigkeit einer Windel, ein Sendemodul zur Übertragung der Messdaten und eine Energieversorgung. Der Feuchtigkeitssensor und der Temperatursensor können in einer flexiblen Hülle des Windelsensors enthalten oder alternativ, lösbar außen an der flexiblen Hülle befestigt sein. Ferner weist der Windelsensor an der Kontaktoberfläche rechteckige Klettelemente auf, die zum Anhaften der flexiblen Hülle an der Windel eingerichtet sind.

EP 1 082 711 A1 offenbart einen Feuchtigkeitsdetektor, wobei die Detektoreinheit in einer Wegwerftasche eingesetzt ist, die einen integrierten Sensorfortsatz aufweist.

Ausgehend von diesem Stand der Technik liegt der Erfindung die Aufgabe zugrunde, eine Vorrichtung zur Überwachung des Zustands einer Einwegwindel mit erhöhter Messgenauigkeit zu schaffen. Zugleich soll trotz vollständiger Wiederverwendbarkeit der Vorrichtung der Reinigungsaufwand minimiert werden.

Diese Aufgabe wird durch eine Vorrichtung mit den Merkmalen des Anspruchs 1 gelöst. Vorteilhafte Ausgestaltungen ergeben sich aus den Merkmalen der Unteransprüche.

Um die Messgenauigkeit zu erhöhen ist zumindest der Feuchtigkeitssensor an einem sich von der Kontaktoberfläche der Hülle nach außen erstreckten Prüfstift angeordnet. Der Stift ist derart eingerichtet, um die Außenhülle der Einwegwindel zu durchsetzen. Der an dem Prüfstift angeordnete Feuchtigkeitssensor befindet sich in Folge dessen in dem von der Außenhülle der Einwegwindel umfassten saugfähigen Material zur Aufnahme von Urin oder Stuhl. Bei dem saugfähigen Material handelt es sich insbesondere um Zellstoffmaterial, das gegebenenfalls mit Absorbern und spezifischen Polymersalzen angereichert ist. Durch den Feuchtigkeitssensor kann eine Änderung der Feuchtigkeit in dem saugfähigen Material schnell und exakt erfasst werden. Der Prüfstift führt in Kombination mit der flexiblen Hülle an der Kontaktfläche damit zu einer verbesserten Messgenauigkeit, ohne die Funktion der Einwegwindel zu beeinträchtigen, weil einerseits der Prüfstift die Verletzung der Außenhülle und andererseits das flexible Material der Hülle den Bereich um die Verletzung der Außenhülle der Einwegwindel abdichtet. Die Haftmittel an dem Windelsensor sorgen für die erforderliche Anhaftung und bündige Anlage an der Außenhülle der Einwegwindel. Für die meisten Einwegwindeln sind Klettverschlüsse als Haftmittel geeignet.

Zugleich ist der Reinigungsaufwand der Vorrichtung reduziert weil lediglich der Prüfstift mit dem saugfähigen Material der Einwegwindel in Kontakt gelangt, während alle weiteren Komponenten geschützt in der flüssigkeitsundurchlässigen Hülle des Windelsensors aufgenommen werden, die außen an der Einwegwindel anliegt.

Das Sendemodul überträgt die erfassten und von der Signalverarbeitungseinheit verarbeiteten Sensordaten über eine Funkstrecke an einen Empfänger. Der Nutzer des Empfängers kann nach Erhalt der verarbeiteten Sensordaten die erforderlichen Pflegemaßnahmen einleiten.

In vorteilhafter Ausgestaltung der Erfindung ist an dem Prüfstift zusätzlich ein Temperatursensor angeordnet. Durch die Messung, sowohl der Feuchtigkeit als auch der Temperatur, lässt sich die Messgenauigkeit weiter erhöhen. Die Temperaturmessung erlaubt es, die Temperaturabhängigkeit des Messsignals des Feuchtigkeitssensors bei der Signalverarbeitung eines resistiven Feuchtigkeitssensors zu berücksichtigen.

Zur effektiven Messung der Temperatur ist der Temperatursensor vorzugsweise an einem freien Ende des Prüfstifts, das heißt insbesondere unmittelbar an dessen Spitze angeordnet. Der Temperatursensor kann beispielsweise im Inneren der Prüfstiftspitze angeordnet sein, während der Feuchtigkeitssensor oberflächennah an dem Prüfstifts angebracht ist. Durch die Anordnung an der Prüfstiftspitze des Kontaktstiftes befindet sich der Temperatursensor in unmittelbarer Nähe zu dem saugfähigen Material der Einwegwindel. Die Temperaturmessung kann beispielsweise auf dem Erfassen einer Widerstandsänderung beruhen. Alternativ kann der Temperatursensor ein thermoelektrisches Element sein, das eine weitgehend temperaturproportionale elektrische Spannung liefert.

Um eine Verletzungsgefahr durch den Prüfstift auszuschließen und den Tragekomfort der Einwegwindel nicht zu beeinträchtigen, weist der Prüfstift in einer vorteilhaften Ausgestaltung der Erfindung einen ersten und einen zweiten Abschnitt auf, wobei der erste Abschnitt sich senkrecht von der Kontaktoberfläche der Hülle nach außen erstreckt, der zweite Abschnitt senkrecht zu dem ersten Abschnitt verläuft und der erste Abschnitt kürzer als der zweite Abschnitt ist.

Die Länge des ersten Abschnitts wird derart gewählt, dass diese deutlich geringer als die Stärke der saugfähigen Lage der Einwegwindel ist und beträgt vorzugsweise zwischen 0,5 cm - 1 cm. Der rechtwinklig dazu verlaufende zweite Abschnitt verläuft im Wesentlichen parallel zu der Kontaktoberfläche der Hülle und damit auch zu der Außenhülle der Einwegwindel. Die Länge des zweiten Abschnitts beträgt vorzugsweise zwischen 1 cm - 2,5 cm.

Der Feuchtigkeitssensor ist vorzugsweise oberflächennah an diesem zweiten Abschnitt angeordnet. Die elektrischen Kontakte des Feuchtigkeitssensors erstrecken sich vorzugsweise durch den Prüfstift bis in die Hülle hinein. Eine gesonderte Verkabelung ist nicht erforderlich. Hierdurch ist gewährleistet, dass die Kontaktierung der Sensoren nicht verunreinigt wird.

Um eine kontaktlose Feuchtigkeitsmessung in dem saugfähigen Material der Einwegwindel zu ermöglichen, ist der Feuchtigkeitssensor vorzugsweise ein kapazitiver Sensor. Ein kapazitiver Sensor weist zwei Elektrodenstrukturen auf, die im Abstand zueinander angeordnet sind. Die elektrische Kapazität zwischen den Elektrodenstrukturen wird stark von der Dielektrizitätszahl εᵣ der Umgebung beeinflusst, insbesondere vom Feuchtigkeitsgehalt des saugfähigen Materials der Einwegwindel. Für die Kapazitätsmessung ist kein direkter Kontakt der Feuchtigkeit mit den Elektrodenstrukturen erforderlich.

Für die Anordnung des kapazitiven Feuchtigkeitssensors an dem Prüfstift ergeben sich daraus unterschiedliche Möglichkeiten: Die Elektrodenstrukturen sind oberflächennah in ein dielektrisches Material des Prüfstiftes eingelassen und das dielektrische Material des Prüfstiftes ist zugleich das Dielektrikum des kapazitiven Feuchtigkeitssensors. Wenn das dielektrische Material ein Kunststoff ist, lässt sich der Prüfstift mit den Elektrodenstrukturen vorteilhaft in einem Arbeitsgang herstellen, beispielsweise im Wege eines Zweikomponentenspritzguss-Verfahrens. Die eingelassen Elektrodenstrukturen sind zugleich durch das Material des Prüfstifts vor Verunreinigungen und Korrosion geschützt bei gleichzeitig guter Reinigungsfähigkeit der homogenen Oberfläche des Prüfstifts aus dem dielektrischen Material.

Die Elektrodenstrukturen können jedoch auch oberflächenbündig in ein dielektrisches Material des Prüfstiftes eingelassen sein, wobei das dielektrische Material des Prüfstiftes zugleich das Dielektrikum des kapazitiven Feuchtigkeitssensors bildet. Zum Schutz der an der Oberfläche des Prüfstifts freiliegenden Elektrodenstrukturen vor Verunreinigungen und Korrosion ist der Prüfstift zusätzlich mit einem dielektrischen Material ummantelt.

In einer weiteren Ausgestaltung der Erfindung dient der Prüfstift als Träger der Elektrodenstrukturen, die auf dessen Oberfläche aus elektrisch isolierendem Material angeordnet sind. Der Prüfstift ist in diesem Fall mit einem dielektrischen Material ummantelt. Der Mantel aus dielektrischem Material bildet zugleich das Dielektrikum zwischen den aufgebrachten Elektrodenstrukturen des kapazitiven Feuchtigkeitssensors und dient dem Schutz der Elektrodenstrukturen vor Verunreinigungen und Korrosion.

Bei sämtlichen Anordnungen des kapazitiven Feuchtigkeitssensors sind dessen Elektrodenstrukturen vollständig vor einem direkten Kontakt mit dem saugfähigen Material der Einwegwindel geschützt.

Das dielektrische Material ist ein Polymer, insbesondere ein organisches Polymer.

Um bei der begrenzten Größe des vorzugsweise kreiszylindrischen Prüfstifts ausreichend große Elektrodenstrukturen des kapazitiven Sensors zu gewährleisten, greifen diese vorzugsweise kammartig ineinander. Der Durchmesser des Prüfstifts beträgt insbesondere zwischen 0,5 cm - 1 cm.

Handelt es sich bei dem Feuchtigkeitssensor um einen Sensor, der eine Änderung der elektrischen Leitfähigkeit in Folge der Durchfeuchtung des saugfähigen Materials in der Einwegwindel als Messgröße erfasst, muss das Material des Prüfstifts bzw. die Ummantelung gegebenenfalls aus einem hygroskopischen Material, insbesondere einem hygroskopischen Polymer bestehen, dessen Leitfähigkeit sich in Abhängigkeit von der Feuchte in der Umgebung ändert. Die Leitfähigkeitsmessung erfolgt insbesondere mittels eines Wechselstroms, beispielsweise bei einer Frequenz von 1 KHz. Die abhängig von der Feuchte gemessenen Impedanzwerte des resistiven Feuchtigkeitssensors sind temperaturabhängig. In diesem Fall ist es empfehlenswert, zusätzlich in jedem Fall auch die Temperatur zu messen und in der Signalverarbeitungseinheit die gemessenen Impedanzwerte temperaturabhängig zu korrigieren.

Um den Sensorstift in Bezug auf die flexible Hülle mechanisch zu stabilisieren, weist die die Signalverarbeitungseinheit aufnehmende Platine innerhalb der Hülle eine Doppelfunktion auf. An dem Prüfstift ist ein in die Hülle hineinragender Befestigungsabschnitt angeordnet, der an der Platine gehaltert ist. Die Platine wird zwischen der oberen und unteren Lage der lediglich an den Rändern verschweißten Hülle oberflächenparallel ausgerichtet und gehalten. Damit wird auch der Befestigungsabschnitt des Prüfstifts, der fest an der Platine gehalten ist, in definierter Lage ausgerichtet, sodass der in Verlängerung des Befestigungsabschnitts verlaufenden erste Abschnitt des Prüfstifts seine im Wesentlichen senkrechte Ausrichtung zur Kontaktoberfläche beibehält.

Um die Platine und den daran befestigten Prüfstift sowie die weiteren Komponenten der Vorrichtung in ihrer Lage zueinander und zur Hülle zu fixieren, ist in einer vorteilhaften Ausgestaltung der Erfindung vorgesehen, dass sämtliche von der Hülle aufgenommen Komponenten der Vorrichtung in eine flexible Gießmasse eingebettet sind. Die Gießmasse kann beispielsweise ein Schmelzklebstoff sein. Unter der Handelsbezeichnung "Technomelt" wird von der Henkel AG & Co. KGaA ein geeigneter Schmelzklebstoff angeboten.

Die Hülle umgibt die vorzugsweise in die flexible Giessmasse eingebetteten Komponenten. Insbesondere kann die Hülle stoffschlüssig mit der Giessmasse verbunden sein, z.B. durch Anspritzen des Materials der Hülle an die flexible Giessmasse.

Die Hülle besteht aus einem chemisch gegen Körperflüssigkeiten sowie Reinigungsmittel, insbesondere Desinfektionsmittel beständigem Material. Als Material kommt insbesondere ein thermoplastisches Elastomer (TPE) in Betracht. Ein geeignetes thermoplastisches Elastomer ist beispielsweise das unter der Handelsbezeichnung verfügbare "THERMOLAST M" der Firma Kraiburg TPE GmbH & Co. KG, das als medizinischer Werkstoff zugelassen und im Wege des Spritzgießen verarbeitbar ist.

Am Übergang zwischen dem Prüfstift und dem Befestigungsabschnitt des Prüfstifts befindet sich eine Dichtung, beispielsweise eine Tülle aus TPE oder Silikon. Hierdurch wird eine gute Abdichtung am Durchgang durch die Hülle erreicht.

In vorteilhafter Ausgestaltung der Erfindung ist vorgesehen, dass eine auf der Kontaktoberfläche angeordnete Dichtoberfläche den Prüfstift ringförmig umgibt und dadurch eine verbesserte Abdichtung der Verletzung der Außenhülle erzielt wird. Die Dichtoberfläche besteht vorzugsweise aus einem dauerelastischen, flüssigkeitsdichten Material.

Wenn in der Hülle des Windelsensors ein Lagesensor angeordnet ist, kann in einer vorteilhaften Ausgestaltung der Erfindung festgestellt werden, ob der Träger ausreichend oft umgelagert wurde. Diese Information ist beispielsweise in der Alten- und Krankenpflege zur Dekubitus-Prophylaxe sinnvoll. Der Lagesensor kann beispielsweise als Beschleunigungssensor in drei Achsen ausgeführt werden. Er ist auch in der Lage, einen Sturz des Windelträgers zu erkennen und das Ereignis über das Sendemodul zu melden.

Zusätzlich kann in der Hülle ein optischer Sensor derart angeordnet sein, dass er über eine transparente, jedoch flüssigkeitsundurchlässige Öffnung in der Kontaktfläche der Hülle einfallendes Licht aufnimmt. Der optische Sensor ist daher, insbesondere in Verbindung mit dem Lagesensor, in der Lage, ein ordnungsgemäßes Anlegen der Vorrichtung zur Überwachung des Zustandes einer Einwegwindel zu kontrollieren. Liegt die Kontaktfläche der Hülle nach korrektem Anlegen an der Außenhülle der Einwegwindel an, fällt über die transparente Öffnung kein oder lediglich eine geringe Menge Licht auf den optischen Sensor ein. Wenn unmittelbar vor dem Anlegen von dem Lagesensor eine für das Anlegen typisches Bewegungsmuster detektiert wurde, spricht dies mit hoher Wahrscheinlichkeit für ein ordnungsgemäßes Anlegen des Windelsensors.

Sofern das Sendemodul ein W-LAN-Modul ist, können die erfassten und verarbeiteten Sensordaten über große Reichweiten übertragen werden. In Verbindung mit einem W-LAN-Modul eignet sich die erfindungsgemäße Vorrichtung mit einer entsprechenden Software daher zu einer revisionssicheren Dokumentation eines ordnungsgemäßen, zeitnahen Wechsels der Einwegwindel sowie gegebenenfalls weiterer Pflegemaßnahmen, wie z.B. eine ordnungsgemäße Umlagerung des Windelträgers. Werden lediglich kürzere Übertragungsstrecken benötigt, kann das Sendemodul auch auf einem anderen Funkstandard, wie beispielsweise dem Bluetooth-Standard basieren, um in Echtzeit die von den Sensoren erfassten Daten zu übertragen.

Um kontaktlos den Akkumulator aufladen zu können, ist im Inneren der Hülle eine Induktionsspule für ein induktives Laden des Akkumulators angeordnet. Alternativ kann ein elektrischer Anschluss für den Akkumulator an der Hülle angeordnet sein, insbesondere ein USB-Anschluss. Aus hygienischen Gründen ist jedoch das induktive Laden zu bevorzugen.

Die Hülle der Vorrichtung besteht vorzugsweise aus TPE oder Silikon. Wenn zusätzlich der Prüfstift mit einem Polymer ummantelt ist oder vollständig aus einem Polymer besteht, ergibt sich eine flüssigkeitsdichte, einfach zu reinigende und desinfizierende Vorrichtung.

Nachfolgend wird die Erfindung anhand der Figuren näher erläutert. Es zeigen
- **Figur 1**: eine perspektivische Ansicht auf eine Kontaktoberfläche einer erfindungsgemäßen Vorrichtung,
- **Figur 2**: eine vergrößerte Darstellung eines ersten Ausführungsbeispiels eines Prüfstifts einer erfindungsgemäßen Vorrichtung sowie
- **Figur 3**: ein zweites Ausführungsbeispiel eines Prüfstifts einer erfindungsgemäßen Vorrichtung.

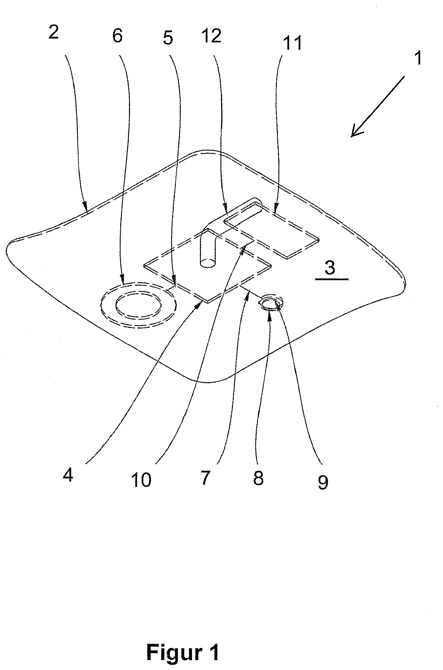

Figur 1 zeigt eine Vorrichtung 1 zur Überwachung des Zustands einer Einwegwindel mit einer Hülle 2 aus flexiblem, flüssigkeitsundurchlässigem Material mit einer Kontaktoberfläche 3. Die Hülle 2 besteht beispielsweise aus PTE oder Silikon und weist eine obere und eine untere Lage auf, die an den Längsrändern miteinander verschweißt sind. Hierdurch ergibt sich eine flache, wenig auftragende Hülle, die sich problemlos mit Hilfe von Haftmitteln mit ihrer Kontaktoberfläche 3 an der Außenhülle der Einwegwindel befestigen lässt. Die Haftmittel können an verschiedenen Stellen oder vollflächig auf der Kontaktoberfläche 3 angebracht sein. Als Haftmittel kommt beispielsweise Klettverschlusselemente in Betracht, die sich mit der Außenhülle der Einwegwindel lösbar verbinden.

In der Hülle 2 sind verschiedene Komponenten der erfindungsgemäßen Vorrichtung 1 untergebracht. Die verschiedenen Komponenten im Inneren der Hülle 2 sind gestrichelt dargestellt. Etwa mittig in der Hülle 2 ist eine Platine 4 mit einer Signalverarbeitungseinheit und einem mit der Signalverarbeitungseinheit verbundenen Sendemodul angeordnet. Auf der Platine 4 befindet sich darüber hinaus ein Akkumulator, der über eine elektrische Verbindung 5 mit einer Induktionsspule 6 verbunden ist. Die Induktionsspule 6 erlaubt ein kontaktloses Aufladen des Akkumulators durch die Hülle 2 hindurch. Des Weiteren ist die Signalverarbeitungseinheit auf der Platine 4 über eine elektrische Verbindung 7 mit einem optischen Sensor 8, zum Beispiel einer Fotodiode, verbunden, die über eine transparente, jedoch flüssigkeitsundurchlässige Öffnung 9 in der Kontaktfläche 3 der Hülle 2 einfallendes Licht aufnimmt.

Über eine weitere elektrische Verbindung 10 ist die Signalverarbeitungseinheit mit einem Lagesensor 11 verbunden, der in dem Ausführungsbeispiel als Beschleunigungssensor in X-, Y- und Z-Richtung ausgestaltet ist.

Schließlich weist die Vorrichtung 1 einen Prüfstift 12 auf, der sich von der Kontaktoberfläche 3 der Hülle 2 nach außen erstreckt, wobei an dem Prüfstift 12 zumindest ein Feuchtigkeitssensor 13 und gegebenenfalls zusätzlich ein Temperatursensor 14 angeordnet ist.

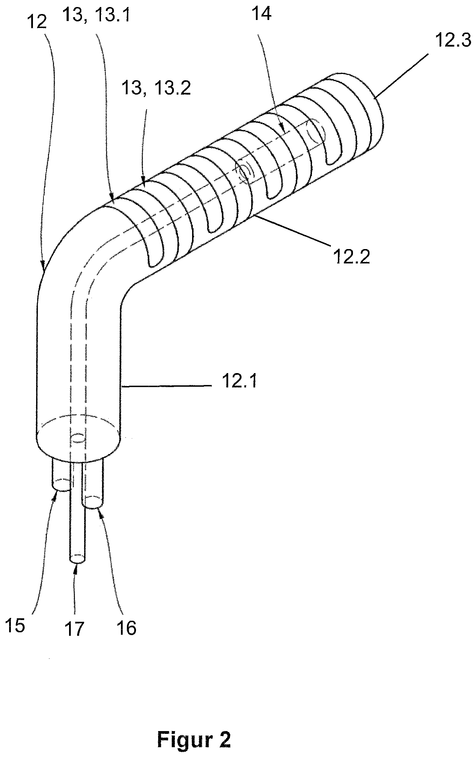

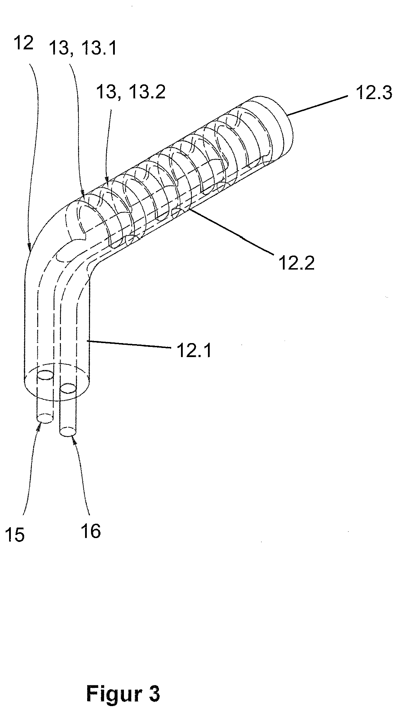

Der im Details in den Figuren 2 und 3 dargestellte Prüfstift 12 weist einen ersten Abschnitt 12.1 und einen zweiten Abschnitt 12.2 auf, wobei der erste Abschnitt 12.1 sich im Wesentlichen senkrecht von der Kontaktoberfläche 3 der Hülle 2 nach außen erstreckt. Der zweite Abschnitt 12.2 verläuft im Wesentlichen senkrecht zu dem ersten Abschnitt 12.1. Der erste Abschnitt 12.1 ist etwa halb so lang, wie der zweite Abschnitt 12.2. Der Querschnitt des Prüfstifts 12 ist rund. Die Spitze 12.3 des Prüfstifts 12 ist leicht sphärisch. Der Durchmesser des Prüfstifts 12 ist derart gewählt, dass mit der Spitze 12.3 die Außenhülle der Einwegwindel problemlos durchdrungen werden kann. Die sphärische Spitze 12.3 sowie die abgewinkelte Ausführung des Prüfstifts 12 trägt dazu bei, dass eine Verletzung des Trägers der Einwegwindel durch den Prüfstift 12 praktisch ausgeschlossen ist und der Tragekomfort der Einwegwindel nicht beeinträchtigt wird.

Um den Prüfstift 12 in seiner Lage zu der flexiblen Hülle 2 zu stabilisieren, ist an dem ersten Abschnitt 12.1 ein in die Hülle 2 hineinragender Befestigungsabschnitt angeordnet, der an der Platine 4 befestigt ist. Aufgrund ihrer flächigen Struktur wird die Platine 4 zwischen der Kontaktoberfläche 3 der Hülle 2 und der gegenüberliegenden Oberfläche der Hülle stabilisiert. Zusätzlich sind die Platine 4 mit dem daran befestigten Prüfstift 12 sowie die weiteren Komponenten der Vorrichtung zur Lagefixierung in eine flexible Giessmasse eingebettet, die von der Hülle aufgenommen wird. Die Hülle kann an die flexible Giessmasse angespritzt sein.

Der Prüfstift nach Figur 2 ist mit einem Feuchtigkeitssensor 13 und einem optionalen Temperatursensor 14 ausgestattet. Der kapazitive Feuchtigkeitssensor 13 weist zwei kammartige, ineinandergreifende Elektrodenstrukturen 13.1, 13.2 auf. Die erste Elektrodenstruktur 13.1 weist einen im Inneren des Prüfstifts 12 geführten elektrischen ersten Kontakt 15 und die zweite Elektrodenstruktur 13.2 einen im Inneren des Prüfstifts 12 geführten zweiten Kontakt 16 auf. Die Kontakte 15, 16 sind mit der Signalverarbeitungseinheit auf der Platine 4 im Inneren der Hülle 2 elektrisch leitend verbunden. Die Elektrodenstrukturen 13.1, 13.2 des kapazitiven Feuchtigkeitssensors 13 sind derart oberflächennah in das Prüfstiftmaterial eingelassen, dass sich die kammartig ineinandergreifenden Elektrodenstrukturen 13.1, 13.2 nicht berühren. Das dielektrische Material des Prüfstifts bildet zugleich das Dielektrikum des kapazitiven Feuchtigkeitssensors 13.

Der Temperatursensor 14 ist im Inneren des Prüfstifts 12 an dessen Spitze 12.3 angeordnet. Hierdurch gelangt der Temperatursensor 14 in die unmittelbare Nähe des saugfähigen Materials der Einwegwindel und erfasst die Temperatur im Wege der Wärmeleitung. Der optionale Temperatursensor 14 ist über Kontakte 17 im Inneren des Prüfstifts 12 mit der Signalverarbeitungseinheit verbunden. Der Temperatursensor dient der zusätzlichen Erfassung der Temperatur des saugfähigen Materials der Einwegwindel und erlaubt daher ebenfalls Rückschlüsse auf etwaige Ausscheidungen. Das Signal des Temperatursensors 14 kann mit dem Signal des Feuchtigkeitssensors abgeglichen werden, um die Messgenauigkeit weiter zu verbessern.

Der Prüfstift 12 nach Figur 3 unterscheidet sich von dem Prüfstift nach Figur 2 lediglich dadurch, dass dieser keinen Temperatursensor 14 aufweist. Des Weiteren wurde der Verlauf der ersten und zweiten Kontakte 15,16 des Feuchtigkeitssensors 13 mit seinen beiden Elektrodenstrukturen 13.1, 13.2 gestrichelt dargestellt. Es ist erkennbar, wie die ineinandergreifenden Zähne der kammartigen Elektrodenstrukturen 13.1, 13.2 abwechselnd jeweils von dem ersten beziehungsweise zweiten Kontakt 15, 16 ausgehen.

Zur Inbetriebnahme der Vorrichtung 1 wird mit der Spitze 12.3 des Prüfstifts 12 die Außenhülle der Einwegwindel an der Stelle durchörtert, an der sich die Flüssigkeit in der Einwegwindel sammelt. Der Prüfstift 12 dringt dabei in die saugfähige Lage der Einwegwindel ein und zwar im Wesentlichen mit horizontaler Ausrichtung des zweiten Abschnitts 12.2 parallel zur Oberfläche der Außenhülle beziehungsweise der daran anliegenden Kontaktoberfläche 3 der Vorrichtung 1. Der erste Abschnitt 12.1 des Prüfstifts weist eine Längserstreckung auf, die kleiner als die Dicke der saugfähigen Lage der Einwegwindel ist.

Kommt es nun zu einer Aufnahme von Urin oder Stuhl in dem saugfähigen Material ändert sich die Kapazität zwischen den Elektrodenstrukturen 13.1, 13.2. Eine etwaige Temperaturabhängigkeit der Messwerte wird durch die von dem Temperatursensor 14 zeitgleich erfasste Temperatur von der Signalverarbeitungseinheit berücksichtigt.

Mit Hilfe des zusätzlich vorgesehenen Lagesensors 11, lassen sich Bewegungen der Vorrichtung 1 feststellen. Insbesondere kann mit Hilfe einer geeigneten Auswertesoftware festgestellt werden, ob der Träger der Einwegwindel lange unverändert liegt, beziehungsweise gestürzt ist.

Ist zusätzlich der optische Sensor 8 vorgesehen, kann ein korrektes Anlegen der Vorrichtung an die Außenhülle der Einwegwindel festgestellt werden. Sobald die Kontaktfläche 3 auf der Außenhülle der Einwegwindel aufliegt, fällt über die transparente Öffnung 9, kein, beziehungsweise weniger Licht auf den optischen Sensor 8. Mit Hilfe des implementierten Lagesensors 11 kann das spezifische Bewegungsmuster zum Anlegen der Vorrichtung 1 an einer Einwegwindel detektiert werden. Sofern in unmittelbarer zeitlicher Abfolge des erkannten Anlegens eine deutliche Reduktion des einfallenden Lichts über die transparente Öffnung 9 von dem optischen Sensor 8 erfasst wird, spricht dies für ein ordnungsgemäßes Anlegen der Vorrichtung 1.

Die vorstehende Beschreibung der erfindungsgemäßen Vorrichtung 1 verdeutlicht, dass diese in der Alten- und Krankenpflege sowie bei der Pflege von Babys vorteilhaft einsetzbar ist. Insbesondere ist es durch das in jede Vorrichtung 1 implementierte Sendemodul möglich, eine Vielzahl von Vorrichtungen 1 an einer zentralen Stelle zu überwachen und die erforderlichen Pflegemaßnahmen zu veranlassen.

### Bezugszeichenliste

| **Nr.** | **Bezeichnung** |
|---|---|
| 1. | Vorrichtung |
| 2. | Hülle |
| 3. | Kontaktoberfläche |
| 4. | Platine |
| 5. | elektrische Verbindung |
| 6. | Induktionsspule |
| 7. | elektrische Verbindung |
| 8. | optischer Sensor |
| 9. | Öffnung |
| 10. | elektrische Verbindung |
| 11. | Lagesensor |
| 12 | Prüfstift |
| 12.1 | erster Abschnitt |
| 12.2 | zweiter Abschnitt |
| 12.3 | Spitze |
| 13 | Feuchtigkeitssensor |
| 13.1 | Elektrodenstruktur |
| 13.2 | Elektrodenstruktur |
| 14 | Temperatursensor |
| 15 | erster Kontakt |
| 16 | zweiter Kontakt |
| 17 | Temperatursensor-Kontakte |

## Patentansprüche

1. Vorrichtung (1) zur Überwachung des Zustands einer Einwegwindel mit einer saugfähiges Material zur Aufnahme von Urin oder Stuhl umfassenden Außenhülle, umfassend
- einen Feuchtigkeitssensor (13),
- eine Signalverarbeitungseinheit zumindest für die Verarbeitung einer Messgröße des Feuchtigkeitssensors (13),
- eine Energieversorgung,
- ein mit der Signalverarbeitungseinheit verbundenes Sendemodul,
- eine Hülle (2) aus flexiblem, flüssigkeitsundurchlässigem Material mit einer Kontaktoberfläche (3), wobei die Signalverarbeitungseinheit, die Energieversorgung und das Sendemodul in der Hülle (2) untergebracht sind,
- einen Prüfstift (12), der sich von der Kontaktoberfläche (3) der Hülle (2) nach außen erstreckt und derart eingerichtet ist, um die Außenhülle der Einwegwindel zu durchsetzen, wobei an dem Prüfstift (12) der Feuchtigkeitssensor (13) angeordnet ist, der sich in Folge des Durchsetzens in dem saugfähigen Material befindet,
- Haftmittel an der Kontaktoberfläche der Hülle (2), die zum lösbaren Befestigen des flexiblen Materials an der Einwegwindel eingerichtet sind, so dass die Hülle (2) außen an der Einwegwindel anliegt, wobei einerseits der Prüfstift (12) die Verletzung der Außenhülle der Einwegwindel und andererseits das flexible Material der Hülle (2) den Bereich um die Verletzung der Außenhülle der Einwegwindel abdichtet und die Haftmittel für die erforderliche Anhaftung und bündige Anlage an der Außenhülle der Einwegwindel sorgen.

2. Vorrichtung nach Anspruch 1, **dadurch gekennzeichnet, dass** an dem Prüfstift (12) zusätzlich ein Temperatursensor (14) angeordnet ist.

3. Vorrichtung nach Anspruch 1 oder 2, **dadurch gekennzeichnet, dass** der Prüfstift (12) einen ersten und einen zweiten Abschnitt (12.1, 12.2) aufweist, der erste Abschnitt (12.1) sich senkrecht von der Kontaktoberfläche (3) der Hülle (2) nach außen erstreckt, der zweite Abschnitt (12.2) senkrecht zu dem ersten Abschnitt (12.1) verläuft und der erste Abschnitt (12.1) kürzer als der zweite Abschnitt (12.2)ist.

4. Vorrichtung nach Anspruch 3, **dadurch gekennzeichnet, dass** der Feuchtigkeitssensor (13) an dem zweiten Abschnitt (12.2) angeordnet ist und sich elektrische Kontakte (15, 16) des Feuchtigkeitssensors (13) durch den Prüfstift (12) bis in die Hülle (2) hinein erstrecken.

5. Vorrichtung nach einem der Ansprüche 1 bis 4 **dadurch gekennzeichnet, dass** der Feuchtigkeitssensor (13) ein kapazitiver Sensor mit zwei Elektrodenstrukturen (13.1, 13.2) ist.

6. Vorrichtung nach Anspruch 5, **dadurch gekennzeichnet, dass** die Elektrodenstrukturen (13.1, 13.2) kammartig ineinandergreifen.

7. Vorrichtung nach Anspruch 5 oder 6, **dadurch gekennzeichnet, dass** die Elektrodenstrukturen (13.1, 13.2) oberflächennah in ein dielektrisches Material des Prüfstiftes (12) eingelassen sind und das dielektrische Material des Prüfstiftes (12) zugleich das Dielektrikum des kapazitiven Feuchtigkeitssensors ist.

8. Vorrichtung nach Anspruch 5 oder 6, **dadurch gekennzeichnet, dass** die Elektrodenstrukturen oberflächenbündig in ein dielektrisches Material des Prüfstiftes eingelassen sind, das dielektrische Material des Prüfstiftes zugleich das Dielektrikum des kapazitiven Feuchtigkeitssensors (13) bildet und der Prüfstift (12) mit einem dielektrischen Material ummantelt ist.

9. Vorrichtung nach Anspruch 5 oder 6, **dadurch gekennzeichnet, dass** die Elektrodenstrukturen (13.1, 13.2) auf der Oberfläche des Prüfstiftes (12) aus einem dielektrischen Material angeordnet sind und der Prüfstift (12) mit einem dielektrischen Material ummantelt ist, das zugleich das Dielektrikum des kapazitiven Feuchtigkeitssensors (13) bildet.

10. Vorrichtung nach einem der Ansprüche 1 bis 9, **dadurch gekennzeichnet, dass** zumindest die Signalverarbeitungseinheit auf einer Platine (4) angeordnet und an dem Prüfstift (12) ein in die Hülle (2) hineinragender Befestigungsabschnitt angeordnet ist, der an der Platine (4) befestigt ist.

11. Vorrichtung nach einem der Ansprüche 1 bis 10, **dadurch gekennzeichnet, dass** in der Hülle (2) ein mit der Signalverarbeitungseinheit verbundener Lagesensor (11) angeordnet ist.

12. Vorrichtung nach einem der Ansprüche 1 bis 11, **dadurch gekennzeichnet, dass** in der Hülle (2) ein mit der Signalverarbeitungseinheit verbundener optischer Sensor (8) derart angeordnet ist, dass der optische Sensor (8) über eine transparente, jedoch flüssigkeitsundurchlässige Öffnung (9) in der Kontaktoberfläche (3) einfallendes Licht aufnimmt.

13. Vorrichtung nach einem der Ansprüche 1 bis 12, **dadurch gekennzeichnet, dass** sämtliche von der Hülle (2) aufgenommen Komponenten der Vorrichtung (1) in eine flexible Gießmasse eingebettet sind und die Hülle (2) stoffschlüssig mit der Giessmasse verbunden ist.

14. Vorrichtung nach einem der Ansprüche 1 bis 13, **dadurch gekennzeichnet, dass** die Haftmittel Klettverschlussmittel umfassen.

15. Vorrichtung nach einem der Ansprüche 1 bis 14, **dadurch gekennzeichnet, dass** die Energieversorgung einen Akkumulator aufweist und eine Induktionsspule (6) für ein induktives Laden des Akkumulators in der Hülle (2) angeordnet ist.

## Claims

1. Device (1) for monitoring the state of a disposable nappy having an outer casing comprising absorbent material for receiving urine or feces, comprising
- a dampness sensor (13),
- a signal processing unit at least for processing a measured variable of the dampness sensor (13),
- an energy supply,
- a transmission module that is connected to the signal processing unit,
- a casing (2) which is made of a flexible, liquid-impermeable material and comprises a contact surface (3), wherein the signal processing unit, the energy supply, and the transmission module are accommodated in the casing (2),
- a test probe (12) which extends outwards from the contact surface (3) of the casing (2) and is configured in such a way as to penetrate the outer casing of the disposable nappy, wherein the dampness sensor (13) is arranged on the test probe (12), which sensor is located in the absorbent material following the puncturing,
- adhesive means on the contact surface of the casing (2), which are configured for detachably fastening the flexible material to the disposable nappy, such that the casing (2) rests on the disposable nappy on the outside, wherein on the one hand the test probe (12) seals the breach of the outer casing of the disposable nappy, and on the other hand the flexible material of the casing (2) seals the region around the breach of the outer casing of the disposable nappy, and the adhesive means ensure the required adhesion and flush contact on the outer casing of the disposable nappy.

2. Device according to claim 1, **characterized in that** a temperature sensor (14) is additionally arranged on the test probe (12).

3. Device according to either claim 1 or 2, **characterized in that** the test probe (12) comprises a first and a second portion (12.1, 12.2), the first portion (12.1) extends perpendicularly outwards from the contact surface (3) of the casing (2), the second portion (12.2) extends perpendicularly to the first portion (12.1), and the first portion (12.1) is shorter than the second portion (12.2).

4. Device according to claim 3, **characterized in that** the dampness sensor (13) is arranged on the second portion (12.2) and electrical contacts (15, 16) of the dampness sensor (13) extend through the test probe (12) as far as into the casing (2).

5. Device according to any of claims 1 to 4, **characterized in that** the dampness sensor (13) is a capacitive sensor comprising two electrode structures (13.1, 13.2).

6. Device according to claim 5, **characterized in that** the electrode structures (13.1, 13.2) mesh with one another in a comb-like manner.

7. Device according to claim 5 or 6, **characterized in that** the electrode structures (13.1, 13.2) are admitted close to the surface into a dielectric material of the test probe (12), and the dielectric material of the test probe (12) is simultaneously the dielectric of the capacitive dampness sensor.

8. Device according to claim 5 or 6, **characterized in that** the electrode structures are admitted in a flush manner into a dielectric material of the test probe, the dielectric material of the test probe simultaneously forms the dielectric of the capacitive dampness sensor (13), and the test probe (12) is encased by a dielectric material.

9. Device according to claim 5 or 6, **characterized in that** the electrode structures (13.1, 13.2) are arranged on the surface of the test probe (12) made of a dielectric material, and the test probe (12) is encased by a dielectric material which simultaneously forms the dielectric of the capacitive dampness sensor (13).

10. Device according to any of claims 1 to 9, **characterized in that** at least the signal processing unit is arranged on a board (4), and a fastening portion, which is fastened on the board (4) and protrudes into the casing (2), is arranged on the test probe (12).

11. Device according to any of claims 1 to 10, **characterized in that** a position sensor (11) which is connected to the signal processing unit is arranged in the casing (2).

12. Device according to any of claims 1 to 11, **characterized in that** an optical sensor (8), which is connected to the signal processing unit, is arranged in the casing (2) in such a way that the optical sensor (8) receives incident light via a transparent but liquid-impermeable opening (9) in the contact surface (3).

13. Device according to any of claims 1 to 12, **characterized in that** all the components of the device (1) received by the casing (2) are embedded in a flexible casting compound, and the casing (2) is integrally bonded with the casting compound.

14. Device according to any of claims 1 to 13, **characterized in that** the adhesive means comprise hook-and-loop fastening means.

15. Device according to any of claims 1 to 14, **characterized in that** the energy supply comprises a rechargeable battery, and an induction coil (6) for inductive charging of the rechargeable battery is arranged in the casing (2).

## Revendications

1. Dispositif (1) de surveillance de l'état d'une couche jetable avec une enveloppe externe comprenant du matériau absorbant pour recevoir de l'urine ou des fèces, comprenant
- un capteur d'humidité (13),
- une unité de traitement de signaux au moins pour le traitement d'une grandeur de mesure du capteur d'humidité (13),
- une alimentation en énergie,
- un module d'envoi relié à l'unité de traitement des signaux,
- une enveloppe (2) composée d'un matériau flexible imperméable à l'humidité avec une surface de contact (3), dans lequel l'unité de traitement de signaux, l'alimentation en énergie et le module d'envoi sont logés dans l'enveloppe (2),
- un bâtonnet de test (12), qui s'étend vers l'extérieur depuis la surface de contact (3) de l'enveloppe (2) et est mis au point de manière à pénétrer l'enveloppe externe de la couche jetable, dans lequel est disposé sur le bâtonnet de test (12) le capteur d'humidité (13), qui se trouve après quoi dans le matériau absorbant,
- des moyens adhésifs sur la surface de contact de l'enveloppe (2), qui sont mis au point pour fixer de manière amovible le matériau flexible sur la couche jetable, si bien que l'enveloppe (2) repose à l'extérieur sur la couche jetable, dans lequel d'une part le bâtonnet de test (12) étanchéifie le dommage de l'enveloppe externe de la couche jetable et d'autre part le matériau flexible de l'enveloppe (2) étanchéifie la zone autour du dommage de l'enveloppe externe de la couche jetable et les moyens adhésifs s'assurent de l'adhésion requise et de l'appui en affleurement contre l'enveloppe externe de la couche jetable.

2. Dispositif selon la revendication 1, **caractérisé en ce qu'**un capteur de température (14) est disposé en supplément sur le bâtonnet de test (12).

3. Dispositif selon la revendication 1 ou 2, **caractérisé en ce que** le bâtonnet de test (12) présente une première et une deuxième section (12.1, 12.2), la première section (12.1) s'étend vers l'extérieur de manière perpendiculaire depuis la surface de contact (3) de l'enveloppe (2), la deuxième section (12.2) s'étend de manière perpendiculaire par rapport à la première section (12.1) et la première section (12.1) est plus courte que la deuxième section (12.2).

4. Dispositif selon la revendication 3, **caractérisé en ce que** le capteur d'humidité (13) est disposé sur la deuxième section (12.2) et des contacts électriques (15, 16) du capteur d'humidité (13) s'étendent à travers le bâtonnet de test (12) jusque dans l'enveloppe (2).

5. Dispositif selon l'une quelconque des revendications 1 à 4, **caractérisé en ce que** le capteur d'humidité (13) est un capteur capacitif avec deux structures d'électrode (13.1, 13.2).

6. Dispositif selon la revendication 5, **caractérisé en ce que** les structures d'électrode (13.1, 13.2) s'imbriquent les unes dans les autres à la manière d'un peigne.

7. Dispositif selon la revendication 5 ou 6, **caractérisé en ce que** les structures d'électrode (13.1, 13.2) sont pratiquées à proximité de la surface dans un matériau diélectrique du bâtonnet de test (12), et le matériau diélectrique du bâtonnet de test (12) est dans le même temps le diélectrique du capteur d'humidité capacitif.

8. Dispositif selon la revendication 5 ou 6, **caractérisé en ce que** les structures d'électrode sont pratiquées en affleurement de surface dans un matériau diélectrique du bâtonnet de test, le matériau diélectrique du bâtonnet de test forme dans le même temps le diélectrique du capteur d'humidité (13) capacitif et le bâtonnet de test (12) est enveloppé d'un matériau diélectrique.

9. Dispositif selon la revendication 5 ou 6, **caractérisé en ce que** le structures d'électrode (13.1, 13.2) sont disposées sur la surface du bâtonnet de test (12) composé d'un matériau diélectrique, et le bâtonnet de test (12) est enveloppé d'un matériau diélectrique, qui forme dans le même temps le diélectrique du capteur d'humidité capacitif (13).

10. Dispositif selon l'une quelconque des revendications 1 à 9, **caractérisé en ce qu'**au moins l'unité de traitement de signaux est disposée sur une platine (4) et une section de fixation dépassant dans l'enveloppe (2), qui est fixée sur la platine (4), est disposée sur le bâtonnet de test (12) .

11. Dispositif selon l'une quelconque des revendications 1 à 10, **caractérisé en ce qu'**un capteur de position (11) relié à l'unité de traitement de signaux est disposé dans l'enveloppe (2).

12. Dispositif selon l'une quelconque des revendications 1 à 11, **caractérisé en ce qu'**un capteur optique (8) relié à l'unité de traitement de signaux est disposé dans l'enveloppe (2) de telle manière que le capteur optique (8) reçoit de la lumière incidente dans la surface de contact (3) par l'intermédiaire d'une ouverture (9) transparente, toutefois imperméable à l'humidité.

13. Dispositif selon l'une quelconque des revendications 1 à 12, **caractérisé en ce que** la totalité des composants, reçus par l'enveloppe (2), du dispositif (1) sont incorporés dans une masse de coulée flexible et l'enveloppe (2) est reliée par liaison de matière à la masse de coulée.

14. Dispositif selon l'une quelconque des revendications 1 à 13, **caractérisé en ce que** les moyens adhésifs comprennent des moyens de fermeture Velcro.

15. Dispositif selon l'une quelconque des revendications 1 à 14, **caractérisé en ce que** l'alimentation en énergie présente un accumulateur et une bobine d'induction (6) est disposée dans l'enveloppe (2) pour une charge par induction de l'accumulateur.
